# EUROPEAN PATENT SPECIFICATION

(11) **EP 0 599 767 B1**
(45) Date of publication and mention of the grant of the patent: **25.10.2000**
(21) Application number: 93500129.7
(22) Date of filing: 10.09.1993
(51) Int. Cl.: A61K 9/20, A61K 31/195

(54) **Process to prepare water-dispersable tablets containing diclofenac**
Verfahren zur Herstellung wasserdispergierbarer Diclofenac enthaltender Tabletten
Procédé pour préparer des comprimés hydro-dispersibles contenant du diclofenac

(30) Priority: 06.10.1992 ES 9201978
(43) Date of publication of application: 01.06.1994
(73) Proprietor: FABRICA ESPANOLA DE PRODUCTOS QUIMICOS Y FARMACEUTICOS, S.A. (FAES), 48940 Leioa-Lamiaco (Vizcaya) (ES)
(72) Inventor: Orjales-Venero, Aurelio, Neguri (Vizcaya) (ES); Beascoa-Alzola, Esperanza, ES-48008 Bilbao (ES); Lucero-De Pablo, Ma Luisa, ES-48990 Algorta (Vizcaya) (ES)
(74) Representative: Flaccus, Rolf-Dieter, Dr.

(56) References cited:
- EP-A- 0 220 805
- EP-A- 0 365 480
- EP-A- 0 408 273
- WO-A-92/10169
- DE-A- 1 492 019
- FR-A- 2 525 474
- DATABASE WPI Week 9215, Derwent Publications Ltd., London, GB; AN 92-105000 & ZA-A-9 000 502 (LOUW) 29 January 1992

## Description

The present invention concerns a dispersible tablet formulation comprising diclofenac. The invention is also directed to a process for the preparation of such a dispersible tablet formulation. The invention also concerns a dispersible tablet formulation for use in administering finely divided diclofenac.

From a. biopharmaceutical point of view, solid pharmaceutical dosage forms, such as tablets and capsules, which are orally administered by swallowing, provide an accurate dosage of the active principle; but, since they have to disintegrate in the gastrointestinal tract prior to their dissolution, the absorption tends to be slower than if the drug is administered in a dispersed form, such as suspensions and powders or granules dispersed in water. In addition, some people are unable or unwilling to swallow tablets and capsules, and thus dipersible tablets are advantageous because are well-accepted specially by children, patients who find the medicine difficult to swallow, the elderly, and patients with mental illness.

The active principle of the dispersible tablets to which this invention refers is diclofenac, a non-steroidal anti-inflammatory compound that also has analgesic properties, in therapeutic doses, usually between 23 and 93 mg per tablet. Its use is indicated in a variety of processes that cause pain and inflammation. In many pharmaceutical specialties, already on the market in numerous countries, it is included as a salt, for example sodium diclofenac or diethylamonium diclofenac. Its usefulness has been proved in long-term therapy of several illnesses with inflammatory component, and it is recommended in the treatment of the rheumatic processes.

The formulation of diclofenac in the form of dispersible tablets is of great interest since, as indicated, diclofenac is useful to treat processes that old people, in particular, suffer from. On the other hand, diclofenac in this galenic form is more rapidly absorbed, and therefore, its therapeutic effect is more quickly perceived. This is very important when dealing with pain.

The European patent number 0 365 480 A1 of Ciba-Geigy AG describes a dispersible formulation of the active principle diclofenac, as well as a procedure for preparing it.

The document DE-A 14 92 019 is concerned with additives for drug tablets in the process of manufacturing such tablets. The granulation step in said process was tried to be avoided by adding to the tablet formulations a mixture of carboxymethylcellulose and at least one of the substances of a group containing polyethylene glycol 6000, together with usual lubricants like stearic acid and magnesium stearate.

The document EP-A 0 408 273 is addressing the field of tabletting compositions of Fosinopril which compositions are described in the prior art as moisture sensitive and only marginally stable and as requiring the use of a protective package in order to allow a practically useful shelf life.

The present invention refers to a new formulation of dispersible diclofenac tablets, that disintegrate in less than 3 minutes after dropped into water at room temperature, and its preparation process. This dispersible tablet in water results in a fine dispersion, which facilitates oral administration of the drug and achieves a good dissolution rate and bioavailability of the active principle.

The present invention concerns a dispersible tablet formulation comprising
- finely divided diclofenac having a particle size of less than 10 µm;
- a disintegrant selected from polyvinylpolypyrrolidone and carboxymethylstarch and combinations thereof;
- a hydrophilic lubricant selected from polyethyleneglycol 6000 and sodium stearylfumarate and combinations thereof;
- one or more of common excipients for solid formulations; and
- colloidal silicon dioxide as a glidant.

The invention also concerns a process for the preparation of a dispersible tablet formulation comprising the steps of
(a) wet granulation of a solid mixture of finely divided diclofenac having a particle size of less than 10 µm, polyvinylpolypyrrolidone and one half of the total amount of carboxymethylstarch in the presence of a granulation liquid consisting of 5 % hydroalcoholic polyethyleneglycol 6000 solution to obtain dry granules containing the active principle;
(b) incorporation of the remainder of said carboxymethylstarch, sodium stearylfumarate, colloidal silicon dioxide and one or more common excipients for solid formulations as a powder to said dry granules to obtain a mixture of granules in a powder; and
(c) compression of said mixture.

The invention also relates to the dispersible tablet formulation as described above for use in administering finely divided diclofenac.

These dispersible tablets can also be used as traditional tablets; in this case, their dispersion in the gastrointestinal tract is also faster than for traditional tablets. Micronized diclofenac, with a particle size of less than 10 µm, is used, thus improving dissolution and bioavailability as compared to tablets in which the active principle is not micronized.

In order to obtain the dispersible tablets referred to in this invention, a compression process is carried out in 3 phases: a) Wet granulation of the active principle and part of the excipients, representing ca. 85% of the total weight of the product to be compressed; b) Incorporation of the rest of the excipients as a pulverulent solid to the dry granules obtained in the first phase; c) compression of the mixture. Excipients have been carefully selected to get a mixture to be compressed with suitable compressibility characteristics, and also so that the tablets obtained be immediately dispersible in water with a satisfactory particle size. These dispersible tablets disintegrate in less than three minmutes when they are subjected to the disintegration test for tablets and capsules as per Appendix XIIA of the 1988 British Pharmacopoeia, and comply with the test for uniformity of dispersion and uniformity of weight.

Common excipients for solid formulations, such as microcrystalline cellulose, corn starch and lactose, are used as diluents to increase the volume of the mass to be compressed and facilitate the compression process.

In order to fulfil the requirements for dispersibility of the tablet when dropped in water, polyvinylpolypyrrolidone and carboxymethylstarch are used in combination as disintegrants; the total amount of disintegrants represents 13 to 17% of the final weight of the tablet.

The disintegrants are incorporated into the mass to be compressed as follows: The polyvinylpoly-pyrrolidone and half of the carboxymethylstarch of the formulation are added before the wet granulation process, in order to promote disintegration directly in the primary particles that make up the granules. The rest of the carbonxymethylstarch is added after the granulation process, blended with the remaining ingredients and then compressed.

In the process to prepare these dispersible tablets conventional hydrophobic lubricants are not used; instead, two hydrophilic products have been selected: Polyethylenglycol 6000, which is added dissolved in the granulation liquid, and sodium stearylfumarate, which is added in a fine powder form before proceeding to compression. Sodium stearylfumarate is preferred over more hydrophobic lubricants because suitable hard tablets of uniform contents, disintegration and dissolution rate are achieved. Sodium stearylfumarate contributes in an important manner to the characteristics of the tablets. The polyethylenglycol 6000 dissolved in the granulation liquid helps to obtain a good quality unabrasive granulated mass, thus causing less deteriotation in the machine used and easier to handle. An important aspect of this invention is the use of these lubricants as indicated.

A glidant can also be added included in the formulation, preferably colloidal silicon dioxide, which provides suitable flow properties to the mixture to be compressed. The glidant is added in the proportion of 0.5% of the final tablets weight.

A flavour and a sweetener are also added in order to provide adequate organoleptic characteristics.

To illustrate the scope of the invention, but without limiting it as there are several possible variations thereof, the following examples are offered.

### Example 1

| Composition per dispersible tablet: | |
|---|---|
| Micronized diclofenac | 46.5 mg |
| Microcrystalline cellulose | 156.5 mg |
| Corn starch | 30 mg |
| Plyethylenglycol 6000 | 8 mg |
| Polyvinylpolypyrrolidone | 15 mg |
| Carboxymethylstarch | 30 mg |
| Sodium saccharine | 4.5 mg |
| Orange flavour | 10 mg |
| Sodium stearylfumarate | 6 mg |
| Colloidal silicon dioxide | 1.5 mg |

### Manufacturing process:

A granulation solution, consisting of a 5% hydroalcoholic polyethylenglycol 6000 solution, is prepared.

The micronized diclofenac, The microcrystalline cellulose, the corn starch, The polyvinylpyrrolidone, and half of the total amount of carboxymethylstarch are mixed, after passed through a 0.6 mm mesh sieve. Once a homogeneous mixture is obtained, it is moistened with the 5% hydroalcoholic polyethylenglycol 6000 solution. The moistened mass is passed through a 1 mm mesh sieve, and the obtained granules are dried and passed through a 0.6 mm mesh sieve. The dry, sieved granules are placed in a suitable mixer, and the flavour, the sweetener and half of the carboxymethylstarch of the formulation, previously passed through a 0.6 mm mesh sieve, are added. Colloidal silicon dioxide and sodium stearylfumarate, previously sieved through 0.6 mm, are added. It is all mixed until a homogeneous mixture is obtained, which is then compressed.

### Example 2

| Composition per dispersible tablet: | |
|---|---|
| Micronized diclofenac | 46.5 mg |
| Microcrystalline cellulose | 178 mg |
| Polyethylenglycol 6000 | 8 mg |
| Polyvinylpolypyrrolidone | 15 mg |
| Carboxymethylstarch | 30 mg |
| Sodium saccharin | 3 mg |
| Orange flavour | 20 mg |
| Sodium stearylfumarate | 6 mg |
| Colloidal silicon dioxide | 1.5 mg |

### Manufacturing process:

The micronized diclofenac, the microcrystalline cellulose, the polyvinylpolypyrrolidone and half of the carboxymethylstarch are mixed. Once a homogeneous mixture is obtanined, it is moistened with the granulation solution, consisting of a 5% hydroalcoholic solution of polyethylenglycol 6000. This is granulated, and the dry granulation is mixed with the sodium saccharin, the orange flavour, the rest of the carboxymethylstarch, the colloidal silicon dioxide, and the sodium stearylfumarate. The mixture thus obtained is compressed.

### Example 3

| Composition per dispersible tablet: | |
|---|---|
| Micronized diclofenac | 46.5 mg |
| Microcrystalline cellulose | 143 mg |
| Lactose | 38.5 mg |
| Polyethylenglycol 6000 | 8 mg |
| Polyvinylpolypyrrolidone | 15 mg |
| Carboxymethylstarch | 35 mg |
| Sodium saccharin | 2 mg |
| Flavour | 17 mg |
| Sodium stearylfumarate | 3 mg |

### Manufacturing process:

The micronized diclofenac, the microcrystalline cellulose, the lactose, the polyvinylpolypyrrolidone, and half of the carboxymethylstarch are mixed. This mixture is moistened with the 5% hydroalcoholic polyethylenglycol 6000 solution. The granulation obtained is dried and then the rest of the carboxymethylstarch, the sodium saccharin, the flavour and lastly the sodium stearylfumarate, are added to it. Once a homogeneous mixture is obtained, it is compressed.

Described the nature of the invention and the way to put it into practice, it should be noted that the foregoing is subject to detail modifications, provided they do not alter its fundamental principle which is characterized by the following claims.

## Claims

1. A dispersible tablet formulation comprising
- finely divided diclofenac having a particle size of less than 10 µm;
- a disintegrant selected from polyvinylpolypyrrolidone and carboxymethylstarch and combinations thereof;
- a hydrophilic lubricant selected from polyethyleneglycol 6000 and sodium stearylfumarate and combinations thereof;
- one or more of common excipients for solid formulations; and
- colloidal silicon dioxide as a glidant.

2. The dispersible tablet formulation according to claim 1, wherein said finely divided diclofenac is present in an amount of from 7 to 23 % by weight.

3. The dispersible tablet formulation according to any of the claims 1 or 2, wherein the total amount of said hydrophilic lubricants is from 2.5 to 5 % by weight.

4. The dispersible tablet formulation according to any of the claims 1 to 3, wherein the total amount of said disintegrants is from 13 to 17 % by weight.

5. A process for the preparation of a dispersible tablet formulation comprising the steps of
(a) wet granulation of a solid mixture of finely divided diclofenac having a particle size of less than 10 µm, polyvinylpolypyrrolidone and one half of the total amount of carboxymethylstarch in the presence of a granulation liquid consisting of 5 % hydroalcoholic polyethyleneglycol 6000 solution to obtain dry granules containing the active principle;
(b) incorporation of the remainder of said carboxymethylstarch. sodium stearylfumarate, colloidal silicon dioxide and one or more common excipients for solid formulations as a powder to said dry granules to obtain a mixture of granules in a powder; and
(c) compression of said mixture.

6. The process according to claims 5, wherein said sodium stearylfumarate is added in an amount of 2.5 to 5 % by weight.

7. The process according to any of the claims 5 or 6, wherein said finely divided diclofenac is added in an amount of from 7 to 23 % by weight.

8. The process according to any of the claims 5 to 7, wherein said colloidal silicon dioxide is added in an amont of 0.5 % by weight.

9. The process according to any of the claims 5 to 8, wherein the total amount of polyvinylpolypyrrolidone and carboxymethylstarch is adjusted to a range of from 13 to 17 % by weight.

10. The dispersible tablet formulation according to any of the claims 1 to 4 for use in administering finely divided diclofenac.

## Patentansprüche

1. Eine dispergierbare Tabletten-Formulierung, welche
- fein verteiltes Diclofenac, das eine Partikelgröße von weniger als 10 µm aufweist;
- ein Zerfallshilfsmittel, ausgewählt aus Polyvinylpolypyrrolidon und Carboxymethyl-Stärke und Kombinationen davon;
- ein hydrophiles Schmiermittel, ausgewählt aus Polyethylenglycol 6000 und Natrium-Stearylfumarat und Kombinationen davon;
- einen oder mehrere gebräuchliche Arzneimittelträger für feste Formulierungen; und
- kolloidales Siliciumdioxid als Gleitmittel
enthält.

2. Dispergierbare Tabletten-Formulierung nach Anspruch 1, dadurch gekennzeichnet, daß besagtes fein verteiltes Diclofenac in einem Anteil von 7 bis 23 Gew.-% enthalten ist.

3. Dispergierbare Tabletten-Formulierung nach einem der Ansprüche 1 oder 2, dadurch gekennzeichnet, daß der Gesamt-Anteil der genannten hydrophilen Schmiermittel 2,5 bis 5 Gew.-% beträgt.

4. Dispergierbare Tabletten-Formulierung nach einem der Ansprüche 1 bis 3, dadurch gekennzeichnet, daß der Gesamt-Anteil der genannten Zerfallshilfsmittel 13 bis 17 Gew.-% beträgt.

5. Ein Verfahren zur Herstellung einer dispergierbaren Tabletten-Formulierung, welches folgende Schritte umfaßt:
(a) Feucht-Granulierung einer Feststoff-Mischung von fein verteiltem Diclofenac, welches eine Partikelgröße von weniger als 10 µm aufweist, Polyvinylpolypyrrolidon und einer Hälfte des Gesamt-Anteils von Carboxymethyl-stärke in Gegenwart einer Granulierungs-Flüssigkeit, bestehend aus 5 %iger wässrig- alkoholischer Polyethylenglycol 6000-Lösung, um trockene Granulatkörner zu erhalten, welche den Wirkstoff enthalten;
(b) Beimengung des Rest-Anteils der besagten Carboxymethyl-Stärke, des Natrium-Stearylfumarates, des kolloidalen Siliciumdioxids und eines oder mehrerer gebräuchlicher Arzneimittelträger für feste Formulierungen als ein Pulver zu den besagten trockenen Granulatkörnern, um ein Gemenge von Granulatkörnern in einem Pulver zu erhalten;
(c) Komprimieren des besagten Gemenges.

6. Verfahren nach Anspruch 5, dadurch gekennzeichnet, daß Natrium-Stearylfumarat in einem Anteil von 2,5 bis 5 Gew.-% beigemengt wird.

7. Verfahren nach einem der Ansprüche 5 oder 6, dadurch gekennzeichnet, daß fein verteiltes Diclofenac in einem Anteil von 7 bis 23 Gew.-% beigemengt wird.

8. Verfahren nach einem der Ansprüche 5 bis 7, dadurch gekennzeichnet, daß das besagte Siliciumdioxid in einem Anteil von 0,5 Gew.-% beigemengt wird.

9. Verfahren nach einem der Ansprüche 5 bis 8, dadurch gekennzeichnet, daß der Gesamt-Anteil von Polyvinylpolypyrrolidon und Carboxymethyl-Stärke auf einen Bereich von 13 bis 17 Gew.-% eingestellt wird.

10. Dispergierbare Tabletten-Formulierung nach einem der Ansprüche 1 bis 4 zur Verwendung zum Verabreichen von fein verteiltem Diclofenac.

## Revendications

1. Une formulation de tablette dispersible comprenant
- du diclofenac finement divisé ayant une largeur de particule de moins de 10 µm;
- une substance désintégrante sélectionnée de polyvinylpolypyrrolidone et d'amidon de carboxymethyle et de leurs combinaisons;
- un lubrifiant hydrophile sélectionné de polyethyleneglycole 6000 et de stearylfumarate de sodium et de leurs combinaisons;
- un ou plusieurs excipients communs pour des formulations solides; et
- du dioxyde de silicone colloïdal comme substance glissante.

2. La formulation de tablette dispersible selon la revendication 1, caractérisé en ce que le dit diclofenac finement divisé est présent dans une quantité de 7 à 23 % en poids.

3. La formulation de tablette dispersible selon quelconque des revendications 1 ou 2, caractérisé en ce que la quantité totale des dits lubrifiants hydrophiles est de 2,5 à 5 % en poids.

4. La formulation de tablette dispersible selon quelconque des revendications 1 à 3, caractérisé en ce que la quantité totale des dites substances désintégrantes est de 13 à 17 % en poids.

5. Un procédé pour la préparation d'une formulation de tablette dispersible comprenant les pas de
(a) la granulation humide d'un mélange solide de diclofenac finement divisé ayant une largeur de particule de moins de 10 µm, de polyvinylpolypyrrolidone et d'une moitié de la quantité totale de l'amidon de carboxymethyle en présence d'une granulation liquide se composant d'une solution à 5 % de polyethyleneglycole 6000 hydroalcoolique pour obtenir des granules secs contenant le principe actif;
(b) l'incorporation du résidu du dit amidon de carboxymethyle, du stearylfumarate de sodium, du dioxyde de silicone colloïdal et d'un ou plusieurs excipients communs pour des formulations solides comme un poudre à dits granules secs pour obtenir un mélange des granules dans un poudre; et
(c) compression du dit mélange.

6. Le procédé selon la revendication 5, caractérisé en ce que dit stearylfumarate de sodium est ajouté dans une quantité de 2,5 à 5 % en poids.

7. Le procédé selon quelconque des revendications 5 ou 6, caractérisé en ce que dit diclofenac finement divisé est ajouté dans une quantité de 7 à 23 % en poids.

8. Le procédé selon quelconque des revendications 5 à 7, caractérisé en ce que dit dioxyde de silicone colloïdal est ajouté dans une quantité de 0,5 % en poids.

9. Le procédé selon quelconque des revendications 5 à 8, caractérisé en ce que la quantité totale de polyvinylpoly-pyrrolidone et d'amidon de carboxymethyle est ajustée à une gamme de 13 à 17 % en poids.

10. La formulation de tablette dispersible selon quelconque des revendications 1 à 4 pour l'utilisation dans l'administration du diclofenac finement divisé.
